# EUROPÄISCHE PATENTSCHRIFT

(11) **EP 2 184 344 B1**
(45) Veröffentlichungstag und Bekanntmachung des Hinweises auf die Patenterteilung: **12.08.2015**
(21) Anmeldenummer: 09013853.8
(22) Anmeldetag: 04.11.2009
(51) Int. Cl.: C12M 3/06

(54) **Mikrofluidischer Bioreaktor**
Microfluidic bioreactor
Bioréacteur microfluidique

(30) Priorität: 05.11.2008 DE 102008056037
(43) Veröffentlichungstag der Anmeldung: 12.05.2010
(73) Patentinhaber: Fraunhofer-Gesellschaft zur Förderung der angewandten Forschung e.V., 80686 München (DE)
(72) Erfinder: Herz, Markus, 80336 München (DE); Hansmann, Jan, 70569 Stuttgart (DE); Holzbauer, Peter, 70374 Stuttgart (DE); Richter, Martin, 81543 München (DE)
(74) Vertreter: Schrell, Andreas

(56) Entgegenhaltungen:
- WO-A-2008/005998
- DE-A-102004 023 053
- US-A- 4 087 327
- US-A1- 2001 034 058
- US-A1- 2003 096 405
- US-B2- 6 379 963
- DATABASE WPI Week 200437 Thomson Scientific, London, GB; AN 2004-396001 XP002570564 & JP 2004 147555 A (ZH SEISAN GIJUTSU KENKYU SHOREIKAI) 27. Mai 2004 (2004-05-27)

## Beschreibung

Die vorliegende Erfindung betrifft Bioreaktoren zur Kultivierung von Zellen und zum Testen von Substanzen sowie Verfahren zur Nutzung dieses Bioreaktors.

Pharmazeutische Wirkstoffe werden häufig mit Hilfe von Gewebekulturen untersucht. Die Qualität der Testergebnisse ist unter anderem davon abhängig, welche Bedingungen in den Zellkulturen während der Durchführung dieser Tests vorliegen. So spielt zum Beispiel die Temperatur eine entscheidende Rolle. Ein möglichst physiologischer und damit vitaler Zustand des Gewebes erfordert in aller Regel weitere, vom Gewebetyp abhängige Kulturbedingungen. Einige Gewebearten benötigen zum Beispiel mechanische Reize in Form von Druck- oder Scherkräften. Zudem ist eine optimale Versorgung mit Nährstoffen von großer Bedeutung. Vor allem bei umfänglich angelegten Screeningstudien zur Identifizierung von Wirkstoffen werden kompakte Systeme benötigt, die diese Funktionen erfüllen können. Die Fähigkeit eines Stoffes, eine bestimmte Zellschicht zu perfundieren, ist ein wichtiger Faktor bei der Wirkstoffbeurteilung. Bei in vitro-Studien werden zur Messung dieses Faktors Diffusionsmessungen durch eine entsprechende Schicht aus Zellen durchgeführt. Dazu trennt eine Schicht der zu untersuchenden Zellen zwei Kammern, wobei eine Kammer als Donor und die andere Kammer als Akzeptor dient. Einfache Aufbauten für Diffusionsmessungen stellen so genannte "Inserts" da. Darin befinden sich die Zellen auf einer künstlichen Membran, die die zwei Messkammern voneinander trennt. Die Umgebungsbedingungen sind hierin rein statisch. Dies entspricht jedoch nicht der in vivo-Situation der Zellen, was die Ergebnisse negativ beeinflussen kann. Eine bessere Möglichkeit der Beurteilung bieten sogenannte Franz-Kammern (Franz, The Journal of Investigative Dermatologes 64 (1975), 190-195). In der Franz-Kammer können beliebige Membranen verwendet werden, was Vorteile gegenüber den Inserts bringt. Auf diesen Membranen befinden sich dann die Zellen und trennen den Donor und den Akzeptor.

Nachteilig an der Franz-Kammer ist, dass diese Kammer einen großen, schwerfälligen und teuren Aufbau mit sich bringt. Darüber hinaus kann die Franz-Kammer nur schwer in einem Klimaschrank oder Inkubator gehandhabt werden und benötigt eine Temperierung mittels Wasser. Dies zieht im Folgenden das Installieren großer externer Pumpen zur Temperierung nach sich. Die Möglichkeit, Zellen auf der Zellmembran Scher- und Druckkräften auszusetzen, besteht mit der Franz-Kammer nicht. Insgesamt ist eine Versuchsanordnung mit der Franz-Kammer nur schwer automatisierbar.

Der vorliegenden Erfindung liegt somit das technische Problem zugrunde, einen Bioreaktor bereitzustellen, der die vorgenannten Nachteile überwindet, insbesondere die Kultivierung von Zellen und besonders die Untersuchung beziehungsweise Identifizierung von Substanzen und Wirkstoffen mit Hilfe von Zellkulturen unter annähernd in vivo-Bedingungen ermöglicht. Der vorliegenden Erfindung liegt weiterhin das technische Problem zugrunde, einen Bioreaktor für die Kultivierung von Zellen und zum Analysieren von Substanzen bereitzustellen, der einen vereinfachten Aufbau und dadurch eine Automatisierung und das Durchführen von Screeningverfahren in verschiedenen parallelen Ansätzen erlaubt.

Dieses technische Problem wird erfindungsgemäß gelöst durch Bereitstellen eines Bioreaktors, umfassend mindestens eine Kulturkammer mit einem Bodenteil und mindestens eine mit der Kulturkammer in Gas- oder Fluidkontakt stehende Mikropumpe.

Erfindungsgemäß ist es somit vorgesehen, einen Bioreaktor bereitzustellen, nämlich einen Bioreaktor umfassend mindestens eine Kulturkammer mit einem Bodenteil, wobei das Bodenteil mindestens eine Öffnung aufweist, wobei der Bioreaktor mindestens zwei durch die .Öffnung miteinander in Gas- oder Fluidkontakt stehende Kulturkammern aufweist, wobei jeder Kulturkammer mindestens eine in Gas- oder Fluidkontakt stehende Mikropumpe zugeordnet ist, wobei die Kulturkammer eine in der Öffnung angeordnete Membran oder einen Filter aufweist.

Im Zusammenhang mit der vorliegenden Erfindung wird unter einem Bioreaktor insbesondere ein Gefäß verstanden, das dazu bestimmt und geeignet ist, biologische Materialien, z. B. Zellen, zu kultivieren. In einer besonders bevorzugten Ausführungsform weist die Kulturkammer ein Bodenteil und Seitenwände sowie optional ein Deckelteil auf. Demgemäß kann in bevorzugter Ausführungsform die Kulturkammer allseitig umschlossen sein, sie kann aber auch insbesondere nach oben hin geöffnet sein.

Ein erfindungsgemäßer Bioreaktor weist erfindungsgemäß zumindest zwei oder mehr Kulturkammern auf, in die das biologische Material, insbesondere die Zellen, eingebracht werden können und wobei jeder Kulturkammer eine Mikropumpe zugeordnet ist. Diese Mikropumpe ist vorzugsweise durch Zu- und Ableitungen mit der Kulturkammer verbunden und ermöglicht es, das in der Kulturkammer für die Kultivierung der Zellen eingesetzte Kulturmedium zu bewegen, insbesondere in besonders fein modulierter, genau dosierter Weise zu bewegen.

Erfindungsgemäß wird es dadurch ermöglicht, physiologische Druck- und Strömungsprofile, die beispielsweise dem Herzschlag nachempfunden sind, durch den Mikroaktor der Pumpe auf die Zellmembran aufzuprägen und dadurch eine Kultivierung von Zellen oder von Gewebe unter physiologischen Bedingungen, beispielsweise durch eine dem Herzschlag nachempfundene Modulation der Förderrate und Druckkurve, zu ermöglichen.

Das der Erfindung zugrunde liegende technische Problem wird auch durch die Bereitstellung von Verfahren zur Kultivierung von Zellen sowie durch ein Verfahren zum Testen von Substanzen unter Verwendung der erfindungsgemäßen Bioreaktoren gelöst.

Im Zusammenhang mit der vorliegenden Erfindung wird unter einer "Mikropumpe" eine Pumpe mit funktionskritischen Abmessungen und/oder funktionskritischen Toleranzen im Mikrometerbereich verstanden. Erfindungsgemäß werden unter funktionskritischen Abmessungen und/oder funktionskritischen Toleranzen der erfindungsgemäßen Mikropumpe Bewegungsspielräume der für die Funktion der Mikropumpe wesentlichen Teile, beispielsweise maximal zurücklegbare Weglängen einer Aktorbewegung, wie auch einer Ventilbewegung, der Mikropumpe verstanden. Erfindungsgemäß bevorzugt sind funktionskritische Abmessungen, zum Beispiel für Aktorbewegungen oder für Ventilbewegungen, von 1 bis 800 µm, bevorzugt von 1 bis 700 µm, bevorzugt von 1 bis 600 µm, insbesondere bevorzugt von 1 bis 500 µm, weiter bevorzugt vom 5 bis 500 µm und insbesondere weiter bevorzugt von 20 bis 500 µm. Unter einer Aktorbewegung kann im Rahmen der vorliegenden Erfindung die Bewegung eines Piezobiegewandlers verstanden werden. Unter einer Ventilbewegung einer Mikropumpe kann z. B. die Öffnungsbewegung eines Klappenventils verstanden werden. In einer bevorzugten Ausführungsform der Erfindung ist vorgesehen, dass die erfindungsgemäße Mikropumpe eine Pumpmembran und zwei passive Klappenventile umfasst. Erfindungsgemäß ist in besonders bevorzugter Ausführungsform vorgesehen, dass die Pumpbewegung der Mikropumpe durch eine entsprechend modulierte elektrische Spannung erzeugt wird. Die elektrische Spannung wird in besonders bevorzugter Ausführungsform durch eine externe Elektronik bereitgestellt. Erfindungsgemäß wird es in besonders bevorzugter Ausführungsform, insbesondere durch den Einsatz der Mikropumpe, möglich, Aktorprinzipien, wie Piezokeramiken einzusetzen. Insbesondere ist die erfindüngsgemäße Mikropumpe in besonders bevorzugter Ausführungsform ein Produkt der Mikrosystemtechnik. Erfindungsgemäß in besonders bevorzugter Ausführungsform weist die Pumpe eine kompakte Baugröße von wenigen Zentimetern, bevorzugt mit einem Durchmesser von 5 bis 60 mm, insbesondere von 10 bis 40 mm und einer Höhe von 3 bis 30 mm, insbesondere von 5 bis 20 mm, auf.

In einer bevorzugten Ausführungsform der Erfindung ist die Mikropumpe eine piezoelektrische Mikromembranpumpe.

In einer bevorzugten Ausführungsform umfasst die piezoelektrische Mikromembranpumpe ein Pumpengehäuse, eine Ventileinheit mit aktiven oder, vorzugsweise, passiven Klappenventilen, vorzugsweise jeweils mindestens ein Einlass- und Auslassventil, und mindestens eine durch mindestens ein piezoelektrisches Element angetriebene Pumpmembran. Das Prinzip der bevorzugt eingesetzten Mikromembranpumpe beruht auf der Deformation eines mit der Membran verbundenen Piezoelementes, z. B. einer Scheibe, Platte, etc.. Beim Anlegen einer Spannung kommt es bevorzugt zu einer Verwölbung der Membran (Biegeeffekt).

In einer bevorzugten Ausführungsform weist die piezoelektrische Mikromembranpumpe Membranventile anstatt oder zusätzlich zu den Klappenventilen auf.

In einer bevorzugten Ausführungsform bestehen die Klappenventile und/oder die Membranventile im Wesentlichen oder allein aus Silizium.

In einer bevorzugten Ausführungsform bestehen die Klappenventile und/oder die Membranventile im Wesentlichen oder allein aus Stahl.

In bevorzugter Ausführungsform besteht das Pumpengehäuse im Wesentlichen oder allein aus Kunststoff.

In einer bevorzugten Ausführungsform der vorliegenden Erfindung beträgt das effektive Hubvolumen der Mikromembranpumpe 100 nl bis 100 µl, insbesondere 500 nl bis 50 µl, insbesondere 1 µl bis 40 µl, insbesondere 10 µl bis 30 µl und in besonders bevorzugter Weise 20 µl bis 25 µl.

In einer bevorzugten Ausführungsform der vorliegenden Erfindung beträgt die Förderrate der Mikromembranpumpe mindestens 5 µl/min, bevorzugt mindestens 100 µl/min, bevorzugt mindestens 500 µl/min und in bevorzugter Weise mindestens 1,2 ml/min.

In einer bevorzugten Ausführungsform der vorliegenden Erfindung ist die Förderrate einstellbar, insbesondere auf 1,2 ml/min einstellbar.

Durch die bevorzugt eingesetzte piezoelektrische Mikromembranpumpe können speziell an die physiologischen Bedingungen angepasste Druckprofile eingestellt werden, da aufgrund der direkten Ansteuerung der Membran und des somit ausgestoßenen Hubvolumens eine kurze Ansprechzeit erreicht wird. Erfindungsgemäß können, insbesondere pulsartige, Druckschwankungen mit einer Frequenz von 0,1 bis 10 Hz, vorzugsweise 0,5 bis 5 Hz, insbesondere 1 Hz, erzielt werden, die vorzugsweise eine rechteckige Amplitudenform aufweisen.

In einer bevorzugten Ausführungsform der Erfindung ist vorgesehen, dass eine Mikropumpe so angeordnet und ausgebildet ist, dass sie eine Strömung erzeugen kann, die parallel zu dem Bodenteil verläuft.

Gemäß der Erfindung ist vorgesehen, dass der Bioreaktor mindestens zwei Kulturkammern, insbesondere übereinander angeordnete Kulturkammern, aufweist und mindestens zwei Mikropumpen aufweist, wobei jeder dieser Kulturkammern eine Mikropumpe zugeordnet ist.

Gemäß der Erfindung ist vorgesehen, dass das Bodenteil der mindestens einen Kulturkammer mindestens eine Öffnung aufweist.

Gemäß der Erfindung ist vorgesehen, dass eine Kulturkammer des Bioreaktors eine in der Öffnung des Bodenteils angeordnete Membran oder einen Filter aufweist.

In einer besonders bevorzugten Ausführungsform weist das Bodenteil dann eine mit einer Membran oder einem Filter versehene Öffnung auf, wenn der Bioreaktor mindestens zwei Kulturkammern aufweist, die übereinander angeordnet sind und wobei die obere Kulturkammer, die die Öffnung im Bodenteil aufweisende Kulturkammer ist und die untere Kulturkammer keine im Bodenteil angeordnete Öffnung aufweist.

Die Erfindung sieht also zumindest zwei verschiedene Arten von Kulturkammern vor, nämlich eine, vorzugsweise als obere Kulturkammer verwendete, Kulturkammer, in deren Bodenteil eine, einen Filter oder eine Membran aufweisende, Öffnung vorgesehen ist und eine andere, vorzugsweise als untere Kulturkammer verwendete, Kulturkammer, deren Bodenteil geschlossen ist. Diese beiden Kulturkammern können gemeinsam miteinander eingesetzt werden, so dass durch die Membran oder den Filter im Bodenteil der oben angeordneten Kulturkammer ein Fluid- oder Gasaustausch zu der darunterliegenden Kulturkammer gewährleistet ist.

In besonders bevorzugter Ausführungsform ist vorgesehen, dass der Bioreaktor mehrere übereinander angeordnete Kulturkammern aufweist, wobei zumindest die am weitesten unten gelegene Kulturkammer in ihrem Bodenteil keine Öffnung aufweist, und wobei zumindest eine der darüberliegenden Kulturkammern eine, bevorzugt mit einer Membran oder einem Filter versehene, Öffnung aufweist, die über diese Öffnung einen Gas- oder Fluidkontakt zu einer darunterliegenden Kulturkammer aufweist. Die Erfindung betrifft daher Bioreaktoren gemäß der vorliegenden Art, enthaltend mindestens eine, ein geschlossenes Bodenteil aufweisende Kulturkammer und mindestens eine weitere, in ihrem Bodenteil eine Öffnung aufweisende, darüberliegende Kulturkammer, in deren Öffnung z. B. eine Membran oder ein Filter angeordnet ist, und wobei allen Kulturkammern, eine Mikropumpe zugeordnet ist.

Demgemäß betrifft die vorliegende Erfindung also auch bevorzugt einen Bioreaktor, umfassend mindestens zwei Kulturkammern, wobei jede Kulturkammer ein Bodenteil aufweist, und mindestens eine obere Kulturkammer, vorzugsweise mehrere obere Kulturkammern, die in Gas- oder Fluidkontakt mit einer unteren Kulturkammer stehen, wobei das Bodenteil der oberen Kulturkammer bzw. der oberen Kulturkammern eine Öffnung und das Bodenteil der unteren Kulturkammer keine Öffnung aufweist, und wobei jeder Kulturkammer, eine Mikropumpe zugeordnet ist.

Es ist vorgesehen, dass der Bioreaktor mindestens zwei miteinander in Gas- oder Fluidkontakt stehende Kulturkammern aufweist, wobei die Membran oder der Filter zwischen den Kulturkammern angeordnet ist und sie so einerseits räumlich trennt, andererseits aber hinsichtlich eines Gas- oder Fluidaustauschs auch verbindet.

In einer besonders bevorzugten Ausführungsform der Erfindung ist vorgesehen, dass der Bioreaktor mindestens zwei durch eine Öffnung miteinander in Gas- oder Fluidkontakt stehende Kulturkammern und mindestens eine in der Öffnung angeordnete Membran umfasst, wobei der Bioreaktor dadurch gekennzeichnet ist, dass jeder Kulturkammer mindestens eine Mikropumpe zugeordnet ist. Erfindungsgemäß ist es daher in bevorzugter Ausführung vorgesehen, einen Bioreaktor bereitzustellen, der zwei separate, durch eine Membran voneinander getrennte Kulturkammern aufweist, wodurch es ermöglicht wird, auf der Membran angeordnete Zellen beziehungsweise Gewebe zu untersuchen, insbesondere Permeationsstudien von Stoffübergängen von der einen in die andere Kulturkammer zu analysieren.

Durch den erfindungsgemäßen Bioreaktor ist es nun möglich, die mindestens zwei Kulturkammern durch die Mikropumpen aktiv zu perfundieren. Unter einer "aktiven Perfusion" wird im Zusammenhang mit der vorliegenden Erfindung insbesondere verstanden, dass durch die mindestens eine Mikropumpe eine Strömung aufgebaut wird, die parallel zu dem Bodenteil und insbesondere bevorzugt parallel zu der in der Öffnung des Bodenteils angeordneten Membran verläuft. Durch einen Strömungsverlauf parallel zu der Membran ist es erfindungsgemäß möglich, auf gezielte Art und Weise das Trägermaterial, insbesondere Zellen, Zellverbände, Gewebe, Gewebesysteme oder Organe, Scherkräften in Form eines "shear flow" oder "shear stress" auszusetzen. Insbesondere ist es möglich, durch die erfindungsgemäße Mikropumpe eine spezielle Modulation der Förderrate und Druckkurve zu erreichen und so physiologische Verhältnisse möglichst genau wiederzugeben, beispielsweise solche Bedingungen, wie sie bei einem Herzschlag vorliegen.

Im Zusammenhang mit der vorliegenden Erfindung werden unter "physiologischen Bedingungen" vorzugsweise Zustände verstanden, wie sie unter anderem in vivo, d. h. insbesondere in Blutgefäßen, Organen oder Kapillaren anzufinden sind. Somit sind unter physiologischen Bedingungen insbesondere bestimmte Strömungsverhältnisse in der Kulturkammer zu verstehen.

Erfindungsgemäß ist es auch vorteilhafterweise möglich, Membranen mit Trägermaterial, beispielsweise Hautgewebe oder auf der Membran kultivierte Zellen, nicht nur in dem erfindungsgemäßen Bioreaktor zu testen, sondern diese bereits in dem Bioreaktor vor der eigentlichen Versuchsdurchführung aufzubauen beziehungsweise zu kultivieren. Im Gegensatz zu der bekannten Franz-Kammer ist es also erfindungsgemäß nicht nötig, die Membran in einem separaten Kulturgefäß vorzukultivieren und vor dem Versuchsbeginn umzusetzen. Dadurch wird vermieden, dass die Membran und/oder die darauf kultivierten Zellen eventuell beschädigt werden.

Im Zusammenhang mit der vorliegenden Erfindung werden unter "Trägermaterial" chemische oder biologische Agenzien verstanden. Erfindungsgemäß sind unter "chemischen oder biologischen Agenzien" vorzugsweise Strukturen zu verstehen, die auf der Membran anhaften. Insbesondere stellen in bevorzugter Ausführungsform Zellen, Zellbestandteile, Bakterien, Bakterienbestandteile, Viren, Virenbestandteile, Proteine sowie Proteinbestandteile und Peptide chemische oder biologische Agenzien dar. Als chemische oder biologische Agenzien sind insbesondere auch Bestandteile der extrazellulären Matrix (ECM), wie Kollagene vom Typ I bis IV, Glycosaminoglykane (GAG), Proteoglykane, Hyaluronsäure, Heparansulfat, Dermatansulfat, Chondroitinsulfat und Keratansulfat, geeignet. Weiterhin sind als chemische oder biologische Agenzien Zelladhäsionsmoleküle (CAMs) wie Immunglobuline, Antikörper, Antikörperfragmente, Cadherine, Lektine, insbesondere Selektine, Integrine, Integrin-bindende Proteine oder Mucine vorgesehen. Unter chemischen oder biologischen Agenzien sind weiterhin Nukleinsäuren, wie DNA oder RNA, Wachstumsfaktoren, Hormone, Chemokine, Cytokine, Rezeptoren, Rezeptorbestandteile, Rezeptor-Liganden, Rezeptor-Antagonisten und Nanopartikel, insbesondere polymere Nanopartikel, sowie geeignete Chemikalien zur Beschichtung der Membran zu verstehen.

Im Zusammenhang mit der vorliegenden Erfindung werden unter Zellen insbesondere eukaryotische und prokaryotische Zellen, insbesondere tierische, pflanzliche, pilzliche oder bakterielle Zellen, sowie Viren, also lebens- und kulturfähige biologische Systeme verstanden. Erfindungsgemäß umfasst der Ausdruck "Zellen" auch Zelllinien, Zellgewebe, Zellkulturen, Gewebe, insbesondere Hautgewebe, Gewebesysteme, Organe oder Organsysteme.

Erfindungsgemäß ist vorgesehen, durch Erzeugen einer Strömung, vorzugsweise parallel zu der Membran, Zellen konkreten Scherkräften auszusetzen. Diese Scherkräfte bewirken, dass auf der Membran anhaftende Zellen aktiviert werden. Diese Aktivierung ermöglicht es erfindungsgemäß unter anderem, dass auf den Zellen befindliche Integrine eine aktive Konformation einnehmen. Diese Konformationsänderung wird durch die sogenannte Mechano-Sensitivität der Integrine bewirkt. Integrine weisen bei vorhandenem "shear stress" eine erhöhte Affinität und Avidität für ihre bekannten ECM-Bindungspartnerproteine auf. Durch die spezifische Wechselwirkung zwischen Integrinen und Proteinen der ECM in Folge von "shear stress" werden verschiedene Signalkaskaden im Inneren der Zelle aktiviert. So erfolgt unter anderem eine Aktivierung verschiedener Kinasen, beispielsweise FAK, c-Src, Fyn sowie verschiedene MAP-Kinasen, wie ERK. Im Gegensatz dazu findet bei Abwesenheit von "shear stress", also ohne das Einwirken von Scherkräften, keine Aktivierung der Integrine statt. So liegen die Integrine unter statischen Bedingungen in einer inaktiven Konformation vor, die eine Wechselwirkung mit Komponenten der ECM und eine Bindung daran nicht oder nur kaum zulässt. Dies bewirkt, dass verschiedene Kinasen spezieller Signaltransduktionswege, wie FAK, c-Src und Fyn, nicht aktiviert sind beziehungsweise in einem inaktiven nichtphosphorylierten Status vorliegen. Neben der Wirkung auf die Konformation von Integrinen beeinflusst "shear stress" darüber hinaus auch die Konformation von verschiedenen Ionen-Kanälen, wie beispielsweise Na⁺-Kanälen oder von K⁺-Kanälen. Weiterhin bewirkt "shear stress" auch eine Regulation von endothelialen lonen-Kanälen. Somit ist das Vorhandensein von "shear stress" ein wesentlicher Faktor für zelluläre Transportereignisse durch eine Aktivierung von Integrinen und in Folge dessen verschiedener Rezeptoren sowie von Transportkanälen. Der erfindungsgemäße Bioreaktor ermöglicht somit, annähernd in vivo-Bedingungen zu erzeugen, in denen insbesondere Zellen unter sogenanntem "shear stress" in einer aktiven, physiologischen Konformation kultiviert beziehungsweise getestet werden können.

Die Erfindung sieht in einer weiteren Ausführungsform einen Bioreaktor vor, der mindestens zwei in Gas- und/oder Fluidkontakt stehende Kulturkammern aufweist, die über eine Öffnung miteinander verbunden sind, und wobei in der Öffnung zumindest eine Membran angeordnet ist und wobei jeder Kulturkammer mindestens eine Mikropumpe so zugeordnet ist, dass diese einen aktiven Transport beziehungsweise eine Strömung von mindestens einer Substanz in einem Gas oder einer Flüssigkeit parallel zu der Membran ermöglicht. Erfindungsgemäß bevorzugt weist ein solcher Bioreaktor weitere Elemente auf, zum Beispiel Zu- und Ableitungen, insbesondere Zu- und Ableitungen von der Kulturkammer zu den Mikropumpen, mindestens eine Vorratskammer für zum Beispiel Kulturmedium, vorzugsweise zwei solcher Vorratskammern, Halterungselemente für die Membran, eine Platte zur Festlegung der Kulturkammergröße und ggf. ähnliches. Erfindungsgemäß ist vorgesehen, dass jeder Kulturkammer eine Mikropumpe zugeordnet ist. In einer weiteren bevorzugten Ausführungsform ist vorgesehen, dass jeder Kulturkammer eine Mikropumpe und eine Vorratskammer zugeordnet sind.

Erfindungsgemäß wird durch die Zuordnung jeweils einer Mikropumpe zu jeweils einer Kulturkammer erreicht, dass in jeder Kulturkammer eigene spezifische Kulturbedingungen, insbesondere Druck- und Flussbedingungen des eingesetzten Kulturmediums, und dadurch auf gegebenenfalls aufgebrachte chemische oder biologische Agenzien, insbesondere Zellen, einwirkende Scherkräfte ausgeübt werden, die individuell in jeder Kulturkammer ein gewünschtes Kulturprofil einstellbar macht.

Im Zusammenhang mit der vorliegenden Erfindung wird unter einem "Kulturprofil" das Einstellen einer bestimmten Fließgeschwindigkeit und eines bestimmten Pumpvolumens des Kulturmediums sowie die Wahl einer Kulturkammer mit einer bestimmten Kulturkammerabmessung, insbesondere Kulturkammerhöhe, verstanden, um dadurch konkrete und genau definierte Scherkräfte auf gegebenenfalls aufgebrachte chemische oder biologische Agenzien, insbesondere Zellen, zu bewirken.

Erfindungsgemäß ist es besonders bevorzugt vorgesehen, dass durch eine Zuordnung jeweils einer Mikropumpe zu jeweils einer Kulturkammer mit in bevorzugter Weise vorgesehenen Zu- und Ableitungen der Mikropumpe zu genau dieser Kulturkammer, die durch die Mikropumpe vorgesehene Druck- und Sogwirkung beziehungweise Scherkräfte primär jeweils die genau dieser Mikropumpe zugeordnete Kulturkammer betrifft. Im Zusammenhang mit der vorliegenden Erfindung ist vorgesehen, dass durch die erfindungsgemäße Zuordnung von jeweils einer Mikropumpe zu jeweils einer Kulturkammer zwei getrennte Kreisläufe aufgebaut werden können. Durch den erfindungsgemäß vorgesehenen Aufbau von zwei getrennten Kreisläufen ist es möglich, eine Strömung, bevorzugt zwei Strömungen, aufzubauen, die parallel zur Membran verläuft. Insbesondere ist erfindungsgemäß vorgesehen, in den Kulturkammern unabhängig voneinander physiologische Bedingungen in Form von speziellen Scherkräften durch ein unterschiedliches Einstellen der jeweils zugeordneten Mikropumpen herzustellen.

In einer bevorzugten Ausführungsform ist vorgesehen, dass die mindestens eine Mikropumpe über eine Zuleitung und eine Ableitung mit der ihr zugeordneten Kulturkammer verbunden ist.

In einer weiteren bevorzugten Ausführungsform ist vorgesehen, dass der mindestens einen Mikropumpe jeweils mindestens eine Vorratskammer zugeordnet ist.

In einer bevorzugten Ausführungsform ist vorgesehen, dass die mindestens eine Mikropumpe des Bioreaktors in einer Mikropumpenkammer angeordnet ist.

Erfindungsgemäß bevorzugt ist der Bioreaktor modular aufgebaut. Im Zusammenhang mit der vorliegenden Erfindung bedeutet der Ausdruck "modular" oder "modularisiert", dass der Bioreaktor als eine Baueinheit aus standardisierten Einheiten, also Modulen der einzelnen Komponenten aufgebaut ist. Der modulare Aufbau ermöglicht es, einzelne Komponenten, wie die Mikropumpe, die Mikropumpenkammer, die Kulturkammer, die Vorratskammer oder eventuell weitere Module, leichter gegen andere Module auszutauschen, ohne in das Gesamtsystem eingreifen zu müssen. Die bevorzugt vorgesehene modulare Bauweise des erfindungsgemäßen Bioreaktors ermöglicht eine flexiblere Betriebsweise und erleichtert das Aufspüren und Beseitigen von Fehlerquellen. Der erfindungsgemäße Aufbau ermöglicht eine individuelle Zusammenstellung einzelner Module je nach gewünschtem Versuchsaufbau. Somit ist es auf einfache Weise möglich, einzelne Module des Reaktors über Schlauchanschlüsse zu verbinden. In einer besonders bevorzugten Ausführungsform der vorliegenden Erfindung ist vorgesehen, dass die fluidischen Anschlüsse, wie etwa Schlauchanschlüsse, bereits in die Module integriert sind. Dies ermöglicht in besonders vorteilhafter Weise, dass durch das Zusammenstecken der einzelnen Module eine fluidische Verbindung zwischen diesen Modulen hergestellt wird, ohne dass weitere externe Schlauchanschlüsse notwendig sind. Der erfindungsgemäß bevorzugte modulare Aufbau ermöglicht es, dass die Mikropumpen verschiedener Kulturkammern über eine einzige, gemeinsame Spannungsquelle versorgt werden können.

Erfindungsgemäß ist in einer bevorzugten Ausführungsform vorgesehen, dass der Bioreaktor sämtliche Module zu dessen Betrieb umfasst. Somit ist erfindungsgemäß bevorzugt vorgesehen, dass nur noch eine Steuerelektronik für das Pumpensystem der Mikropumpen angeschlossen werden muss. Der erfindungsgemäße modulare Aufbau stellt damit eine deutliche Vereinfachung gegenüber dem vergleichsweise aufwendigen Aufbau der Franz-Kammer dar. Insbesondere ist es ein Vorteil des erfindungsgemäßen modularen Aufbaus, dass dadurch mehrere Reaktoren parallel zueinander betrieben werden können.

In einer besonders bevorzugten Ausführungsform ist vorgesehen, dass die Kulturkammern jeweils in Form eines Moduls ausgeführt sind.

In einer weiteren bevorzugten Ausführungsform ist vorgesehen, dass die Mikropumpenkammern jeweils in Form eines Moduls ausgeführt sind.

In einer besonders bevorzugten Ausführungsform ist vorgesehen, dass die Vorratskammer in Form eines Moduls ausgeführt ist.

Bevorzugt ist also vorgesehen, dass die Kulturkammern, die Mikropumpenkammern und die mindestens eine Vorratskammer jeweils in Form eines Moduls ausgeführt sind.

In einer bevorzugten Ausführungsform der Erfindung ist vorgesehen, dass die Module quaderförmig sind.

Erfindungsgemäß bevorzugt sind die Außenabmessungen der als Module ausgeführten Kulturkammern, der Mikropumpenkammern und der mindestens einen Vorratskammer identisch.

Der besonders kompakte Aufbau des erfindungsgemäß bereitgestellten Bioreaktors ermöglicht es, viele solcher Bioreaktoren parallel zu betreiben und diese auch in umfänglichen parallelen Studien einzusetzen. Insbesondere ermöglicht der erfindungsgemäße Bioreaktor die Aufrechterhaltung von physiologischen Flüssen gesteuert durch die Mikropumpen. Auf diese Weise ist es möglich, auf der Membran kultivierte Zellen unter physiologischen Bedingungen zu kultivieren und nahezu in vivo-Bedingungen herzustellen, um realistischere Permeationskonstanten zu erhalten.

In einer bevorzugten Ausführungsform der Erfindung ist vorgesehen, dass auf der Membran chemische oder biologische Agenzien aufgebracht sind.

In einer weiteren bevorzugten Ausführungsform der Erfindung ist vorgesehen, dass die Membran eine beschichtete künstliche Membran oder eine biologische Membran ist.

In einer bevorzugten Ausführungsform der Erfindung ist vorgesehen, dass die künstliche Membran aus einem Material besteht, das ausgewählt ist aus der Gruppe bestehend aus Polycarbonat, Polymethylen (PE) und Polyethylenterephthalat (PET).

In einer weiteren bevorzugten Ausführungsform der Erfindung ist vorgesehen, dass die biologische Membran ausgewählt ist aus der Gruppe bestehend aus Dünndarm und Kollagen. Erfindungsgemäß bevorzugt besteht die biologische Membran aus Dünndarm aus Schweinen oder Lagen (sheets) aus Kollagen.

Ein weiterer Gegenstand der Erfindung ist ein Verfahren zum Kultivieren von Zellen, insbesondere also auch Gewebe, Bakterien, Pilzen etc., wobei die Zellen, also zum Beispiel Gewebe, Bakterien, Pilze, Zellen etc., in einer Kulturkammer eines erfindungsgemäßen Bioreaktors aufgebracht und die Zellen unter aktiver Perfusion der Kulturkammer mit dem Kulturmedium durch eine Strömung parallel zu der Membran kultiviert werden.

In einer bevorzugten Ausführungsform des erfindungsgemäßen Verfahrens zum Kultivieren von Zellen sind die Zellen auf der Membran der Kulturkammer aufgebracht.

In einer besonders bevorzugten Ausführungsform kann vorgesehen sein, die in dem genannten erfindungsgemäßen Kultivierverfahren vorgesehene Membran zusätzlich zu den aufgebrachten Zellen mit einem erfindungsgemäß eingesetzten Trägermaterial, insbesondere mit chemischen oder biologischen Agenzien zu versehen, insbesondere zu beschichten.

In besonders bevorzugter Ausführungsform kann vorgesehen sein, das oder die chemischen oder biologischen Agenzien lediglich auf einer Seite anzubringen, vorzugsweise zu beschichten.

In einer weiteren bevorzugten Ausführungsform kann vorgesehen sein, das oder die chemischen oder biologischen Agenzien auf beiden Seiten der Membran anzubringen, vorzugsweise zu beschichten.

In besonders bevorzugter Weise kann vorgesehen sein, dass diese Beschichtung eine Adhäsion der Zellen ermöglicht oder verbessert.

In einer bevorzugten Ausführungsform der Erfindung ist vorgesehen, dass die Membran nur auf einer Seite mit Zellen beschichtet ist. Somit ist erfindungsgemäß vorgesehen, die Membran entweder nur auf der oberen Seite oder nur auf der unteren Seite mit Zellen zu beschichten.

In einer weiteren bevorzugten Ausführungsform der Erfindung ist vorgesehen, dass die Membran auf beiden Seiten, nämlich auf der oberen Seite sowie auch auf der unteren Seite, einheitlich mit Zellen beschichtet ist.

In einer bevorzugten Ausführungsform der Erfindung ist vorgesehen, dass die Membran auf beiden Seiten, nämlich auf der oberen Seite und auf der unteren Seite, unterschiedlich mit Zellen beschichtet ist.

In einer weiteren Ausführungsform der Erfindung ist vorgesehen, zunächst Zellen auf der oberen Seite der Membran zu beschichten und nach einer Transmigration der Zellen auf die untere Seite der Membran die obere Seite der Membran mit weiteren Zellen zu beschichten.

Erfindungsgemäß ist vorgesehen, dass die von der oberen Seite auf die untere Seite der Membran migrierten Zellen gleich oder unterschiedlich zu den danach auf der oberen Seite der Membran beschichteten Zellen sind.

Ein weiterer Vorteil des erfindungsgemäßen Verfahrens im Vergleich zu einer Kultivierung in Inserts besteht darin, dass in dem erfindungsgemäßen Verfahren Zellen auf verschiedenen Trägerstrukturen als Membranen kultiviert werden können. So ist es möglich, neben beschichteten künstlichen Membranen auch biologische Membranen zu verwenden. Dies bedeutet einen entscheidenden Vorteil für die Qualität der durchgeführten Untersuchungen, da nicht auf die vorgegebene künstliche Membran von Inserts zurückgegriffen werden muss. Vielmehr besteht durch das erfindungsgemäße Verfahren nunmehr die Möglichkeit, die Art der Membran individuell an die gewünschte Kultivierung der Zellen und gegebenenfalls an einen anschließenden Versuch oder eine Untersuchung anzupassen.

Ein weiterer Gegenstand der vorliegenden Erfindung ist ein Verfahren zum Testen von mindestens einer Substanz, wobei die zu testende Substanz in einer Kulturkammer eines erfindungsgemäßen Bioreaktors eingebracht und dessen Wirkung auf die in der Kulturkammer des Bioreaktors befindlichen chemischen oder biologischen Agenzien, insbesondere Zellen, untersucht wird.

In einer bevorzugten Ausführungsform ist ein Verfahren zum Testen von mindestens einer in dem Kulturmedium enthaltenen Substanz vorgesehen, wobei die Zellen auf einer Membran des Bioreaktors aufgebracht werden und die Wirkung der zu testenden Substanz besonders bevorzugt unter aktiver Perfusion der Kulturkammer mit dem diese Substanz enthaltenden Kulturmedium durch eine Strömung parallel zu der Membran untersucht wird.

Durch das erfindungsgemäße Verfahren zum Testen wird es ermöglicht, eine Substanz unter annähernd in vivo-Bedingungen in Form von Permeationsstudien zu untersuchen. Die erfindungsgemäß vorgesehene Kultivierung unter Einfluss der Mikropumpen bewirkten Scherkräfte führt zu einer Expression bestimmter Proteine, wie Rezeptoren, auf der Zelloberfläche. Eine zu testende Substanz kann dadurch erfindungsgemäß von einer auf der Membran aufgebrachten Zelle gegebenenfalls besser aufgenommen werden, wie dies unter statischen Bedingungen, also bei Fehlen von "shear stress", der Fall wäre.

Das erfindungsgemäße Verfahren ermöglicht es in besonders vorteilhafter Weise, die Scherkräfte auf die Membran in Abhängigkeit von der Fließgeschwindigkeit zu variieren.

Bedingungen, die die Perfusion der Kulturkammer beeinflussen, sind neben der Fließgeschwindigkeit der Flüssigkeit oder des Gases parallel über die Membran auch die Geometrie der Kulturkammern, insbesondere die Höhe einer oder beider Kulturkammern, die Art der Membran, die Größe der durch die Membran abgedeckten Öffnung zwischen den Kulturkammern, die Viskosität und Art des Kulturmediums, die Art der aufgebrachten Zellen beziehungsweise Gewebe etc. Insbesondere ist es durch Wahl einer geeigneten Geometrie, insbesondere der Höhe der Kulturkammern möglich, die Perfusionsrate durch die Membran zu ändern. Gleiches lässt sich durch Variation der anderen Flussbedingungen des Mediums durch die Membran erreichen.

In einer bevorzugten Ausführungsform der Erfindung ist vorgesehen, dass die durch die aktive Perfusion bewirkten Scherkräfte auf die Membran in Abhängigkeit von der Fließgeschwindigkeit durch die Wahl einer geeigneten Kulturkammerhöhe und der Flussbedingungen im Bioreaktor verändert werden.

In einer bevorzugten Ausführungsform ist vorgesehen, dass die zu testende Substanz als wässrige Lösung oder als in Luft verstäubte Substanz vorliegt. In dem erfindungsgemäßen Verfahren ist es entsprechend möglich, sowohl wässrige Lösungen als auch in Luft verstäubte Wirkstoffe jeweils in der Kulturkammer über die Membran zu leiten.

In einer weiteren Ausführungsform ist vorgesehen, dass die zu testende Substanz ein Wirkstoff ist. Im Zusammenhang mit der vorliegenden Erfindung wird unter einem Wirkstoff eine Zusammensetzung verstanden, die einen Effekt auf biologische oder chemische Agenzien, insbesondere Zellen oder Teile davon, insbesondere auf Zellorganellen oder zelluläre Komponenten, aufweist. Der durch den Wirkstoff vermittelte Effekt induziert durch eine direkte oder indirekte Interaktion mit zellulären Makromolekülen eine Anzahl von funktionellen Änderungen. Insbesondere ist unter einem Wirkstoff eine Zusammensetzung mit diagnostischer oder therapeutischer Wirkung zu verstehen.

In einer bevorzugten Ausführungsform ist vorgesehen, durch Einbringen mindestens einer zu testenden Substanz in eine erste Kulturkammer des Bioreaktors und durch Messen der durch Permeation resultierenden Konzentration der zu testenden Substanzen in einer zweiten Kulturkammer des Bioreaktors die Permeabilität der Membran zu bestimmen.

Somit ist es erfindungsgemäß möglich, Permeationsstudien von Stoffübergängen von einer Kulturkammer durch die Membran, und gegebenenfalls darauf angebrachte biologische oder chemische Agenzien, insbesondere Gewebe oder Zellen, in eine zweite Kulturkammer durchzuführen.

Weitere Ausführungsformen der Erfindung ergeben sich aus den Unteransprüchen.

Die vorliegende Erfindung wird anhand der nachstehenden Beispiele und Figuren näher erläutert. Die Beispiele sind nicht beschränkend zu verstehen.
Figur 1 zeigt eine schematische Darstellung eines nicht erfindungsgemäßen Bioreaktors mit einer Mikropumpe und einer Kulturkammer.
Figur 2 zeigt eine schematische Darstellung eines erfindungsgemäßen Bioreaktors mit zwei Kulturkammern und jeweils einer Kulturkammer zugeordneten Mikropumpen.
Figur 3 zeigt eine schematische Darstellung eines erfindungsgemäßen Bioreaktors mit zwei Vorratskammern, zwei Kulturkammern und einer Mikropumpenkammer.
Figur 4 zeigt eine Detailansicht eines Bioreaktors nach Figur 3.
Figur 5 zeigt einen Querschnitt durch einen Bioreaktor gemäß Figur 3.
Figur 6 zeigt einen Querschnitt durch einen Teil des Bioreaktors gemäß Figur 4.
Figur 7 zeigt perspektivisch den Aufbau eines Teils des Bioreaktors gemäß Figur 4.

### Beispiel 1

### Aufbau eines nicht erfindungsgemäßen Bioreaktors

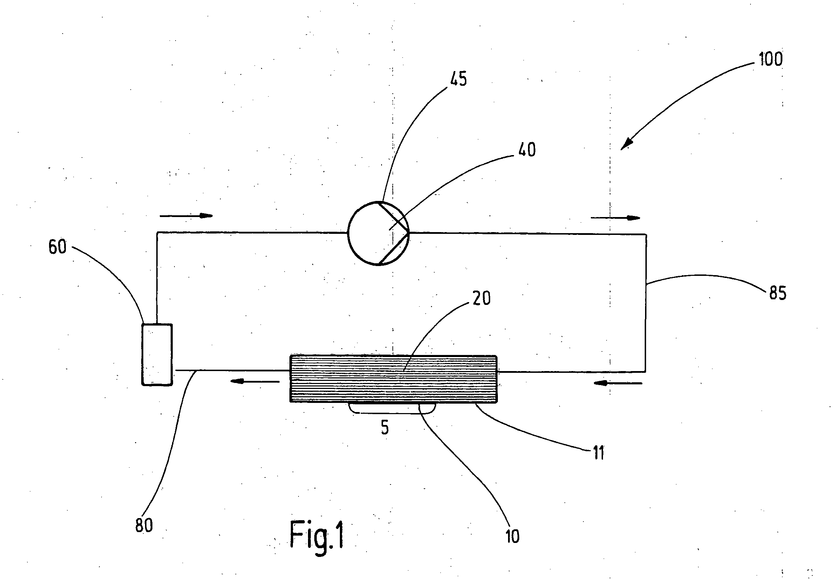

Figur 1 zeigt einen nicht erfindungsgemäßen Bioreaktor (100) mit einer Kulturkammer (20), einer der Kulturkammer (20) zugeordneten Mikropumpe (40), wobei die Mikropumpe eine kompakte Bauweise mit einem Durchmesser von 10 bis 40 mm und einer Höhe von 5 bis 20 mm aufweist, einer diese Mikropumpe (40) enthaltende Mikropumpenkammer (45), einem Bodenteil (11) mit einer Öffnung (5), einer in die Öffnung (5) des Bodenteils (11) angeordneten Membran (10), einer Vorratskammer (60) sowie einer Zuleitung (85) und einer Ableitung (80) für die Zu- und Abfuhr von beispielsweise Kulturmedium in die Kulturkammer (20). In einer erfindungsgemäßen Funktionsweise des Bioreaktors (100) ist vorgesehen, dass Gas und/oder Fluid aus der Vorratskammer (60) durch die Mikropumpe (40), die in der Mikropumpenkammer (45) angeordnet ist, durch die Zuleitung (85) in die Kulturkammer (20) gepumpt wird und durch die Ableitung (80) zurück in die Vorratskammer (60) fließen kann. Der Strömungsverlauf stellt sich als parallel zu dem Bodenteil (11) gerichtet dar.

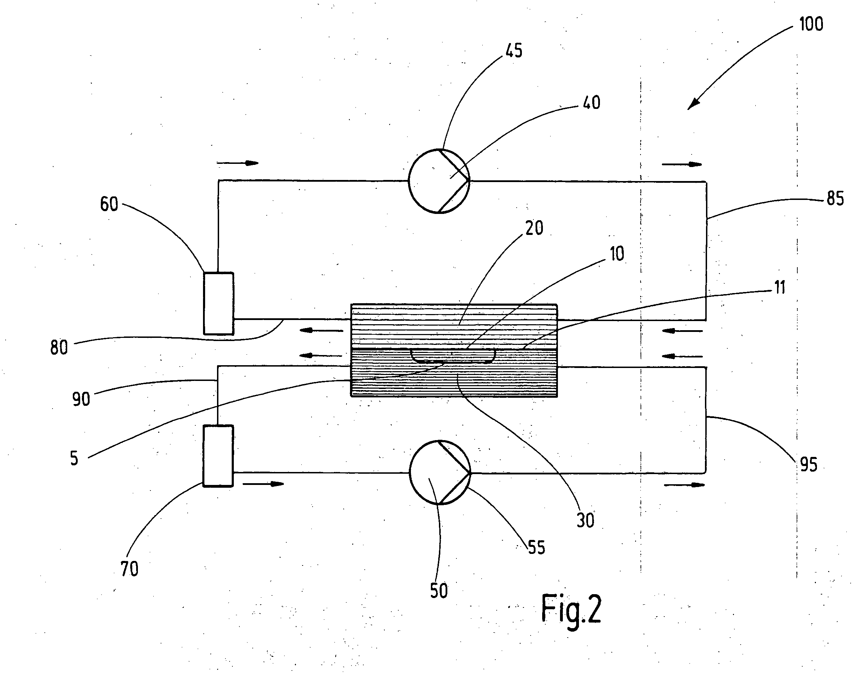

Figur 2 zeigt einen erfindungsgemäßen Bioreaktor (100) mit einer modularen, oberen im Bodenteil (11) eine Öffnung (5) aufweisenden Kulturkammer (20), einer modularen unteren, nach oben hin geöffneten Kulturkammer (30) mit geschlossenem Bodenteil, einer der oberen Kulturkammer (20) zugeordneten Mikropumpe (40) in einer Mikropumpenkammer (45), einer der unteren Kulturkammer (30) zugeordneten Mikropumpe (50) in einer Mikropumpenkammer (55), einem Bodenteil (11) der oberen Kulturkammer (20) mit einer Öffnung (5), einer in die Öffnung (5) des Bodenteils (11) angeordneten Membran (10), die einen Gas- und/oder Fluidkontakt zwischen der oberen Kulturkammer (20) und der unteren Kulturkammer (30) ermöglicht, einer der oberen Kulturkammer (20) zugeordnete Vorratskammer (60), die durch eine Zuleitung (85) und durch eine Ableitung (80) die Zu- und Abfuhr von beispielsweise Kulturmedium in die obere Kulturkammer (20) ermöglicht und einer der unteren Kulturkammer (30) zugeordneten Vorratskammer (70), die durch eine Zuleitung (95) und eine Ableitung (90) die Zu- und Abfuhr von beispielsweise Kulturmedium in die untere Kulturkammer (30) ermöglicht. In einer erfindungsgemäßen Funktionsweise des Bioreaktors (100) wird Gas und/oder Fluid aus der Vorratskammer (60) durch die in der Mikropumpenkammer (45) angeordnete Mikropumpe (40) durch die Zuleitung (85) in die obere Kulturkammer (20) gepumpt und fließt durch die Ableitung (80) zurück zur Vorratskammer (60). Gleichermaßen wird Gas und/oder Fluid aus der unteren Vorratskammer (70) über die Zuleitung (95) durch die in der Mikropumpenkammer (55) angeordnete Mikropumpe (50) in die untere Kulturkammer (30) gepumpt, woraus das Gas und/oder Fluid über die Ableitung (90) zurück in die Vorratskammer (70) fließt. Die beiden Kulturkammern, die über die Membran (10) miteinander in Gas- und/oder Fluidkontakt stehen, werden also getrennt voneinander durch jeweils eine Mikropumpe (40,50) separat einstellbaren Strömungsverhältnissen ausgesetzt, wobei die Strömung im Wesentlichen jeweils parallel zu dem Bodenteil verläuft.

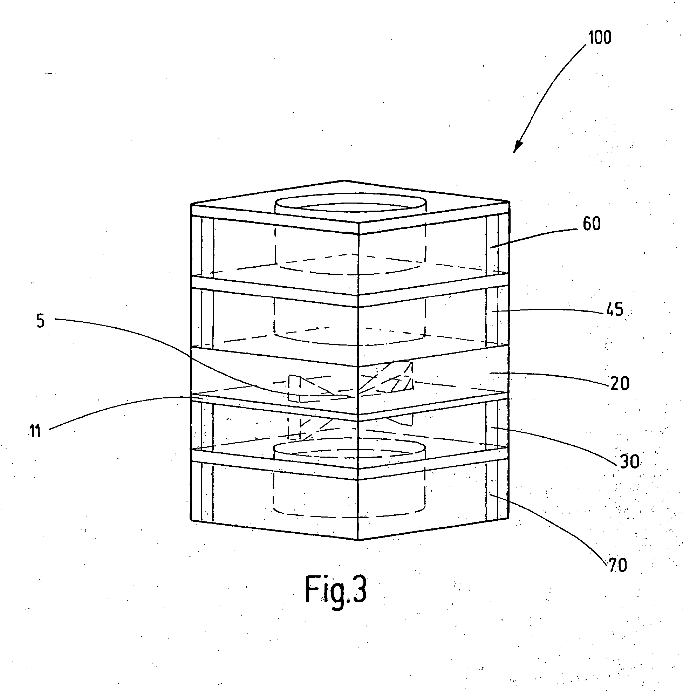

Figur 3 zeigt eine schematische Darstellung des modularen Aufbaus des Bioreaktors (100) mit einer oberen, modularen Kulturkammer (20), mit einem eine Öffnung (5) aufweisenden Bodenteil (11), wobei in die Öffnung (5) eine nicht dargestellte Membran angeordnet werden kann, mit einer unteren, modularen Kulturkammer (30), eine der oberen Kulturkammer (20) zugeordnete, modulare Vorratskammer (60), eine zwischen dieser Vorratskammer (60) und der oberen Kulturkammer (20) liegende Mikropumpenkammer (45) sowie eine der unteren Kulturkammer (30) zugeordnete, modulare Vorratskammer (70), wobei die obere Vorratskammer (60) an die Mikropumpenkammer (45) und diese an die obere Kulturkammer (20) und wobei die untere Vorratskammer (70) an die untere Kulturkammer (30) durch nicht dargestellte, in den Modulen integrierte fluidische Anschlüsse angeschlossen ist. In der Darstellung ist der modulare Aufbau durch eine quaderförmige Ausgestaltung der oberen Vorratskammer (60), der Mikropumpenkammer (45), der oberen Kulturkammer (20), der unteren Kulturkammer (30) sowie der unteren Vorratskammer (70) dargestellt.

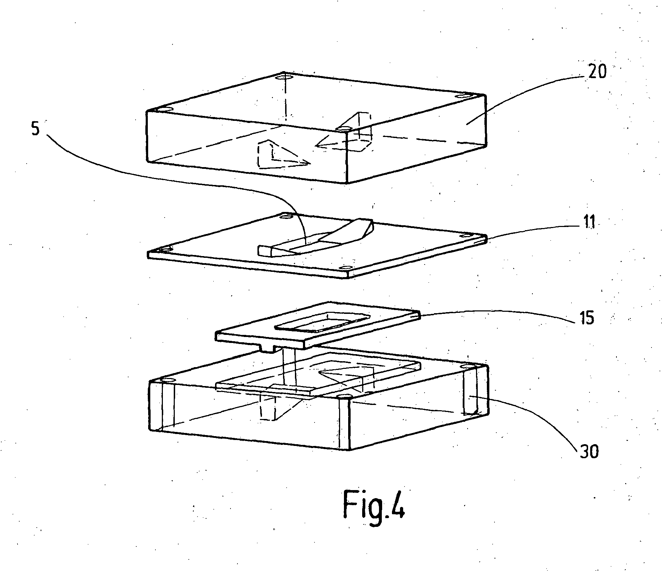

Figur 4 zeigt eine schematische Darstellung des modularen Aufbaus mit einer oberen Kulturkammer (20), die quaderförmig ausgebildet ist, mit einem Bodenteil (11) mit im Vergleich zu der Grundfläche der oberen Kulturkammer (20) identischer Fläche, wobei das Bodenteil (11) eine Öffnung (5) aufweist, in die die Halterung (15) für die nicht dargestellte Membran eingepasst werden kann, mit einer unteren Kulturkammer (30), die ebenfalls quaderförmig ausgebildet ist und identische Abmessungen besitzt wie die obere Kulturkammer (20).

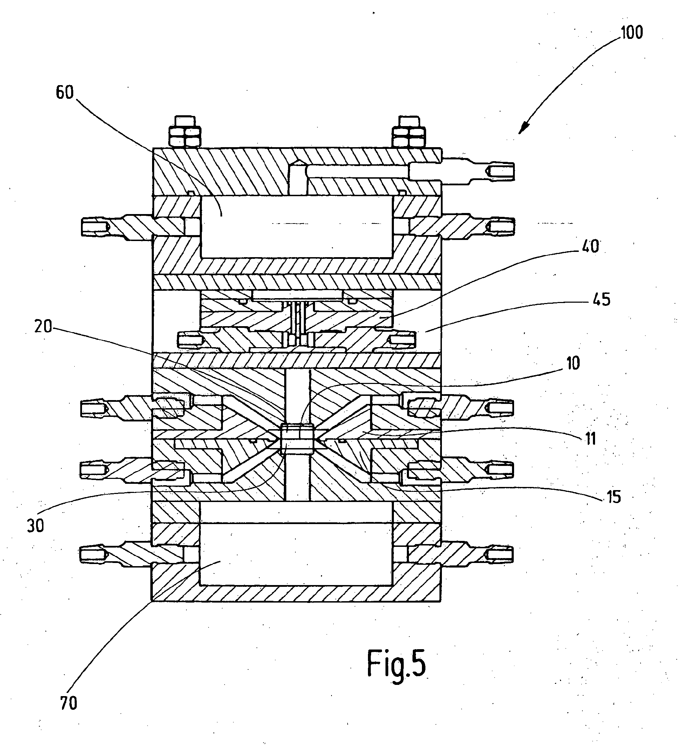

Figur 5 zeigt eine weitere Darstellung des erfindungsgemäßen Bioreaktors (100) im Querschnitt, in dem die obere Vorratskammer (60), die Mikropumpenkammer (45) mit der Mikropumpe (40), das Bodenteil (11), die obere Kulturkammer (20), die untere Kulturkammer (30) und die untere Vorratskammer (70) eine gleiche Grundfläche aufweisen. In das Bodenteil (11) ist die Halterung (15) für die Membran (10) angeordnet.

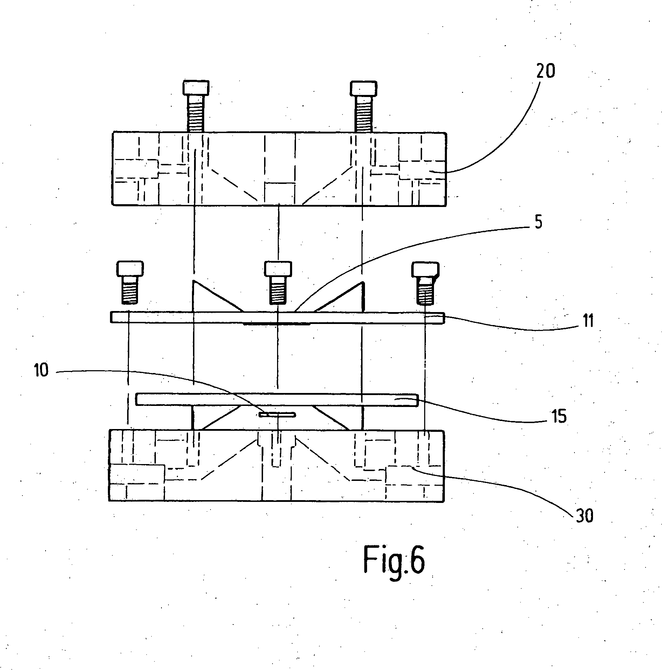

Figur 6 zeigt in einem weiteren Querschnitt den modularen Aufbau einer oberen Kulturkammer (20), eines Bodenteils (11) mit einer Öffnung (5) sowie einer Halterung (15) für die Membran (10) sowie eine untere Kulturkammer (30), in die die Halterung (15) für die Membran (10) eingepasst werden kann. Die obere Kulturkammer (20), das Bodenteil (11) sowie die untere Kulturkammer (30) weisen eine gleiche Breite bzw. Länge auf.

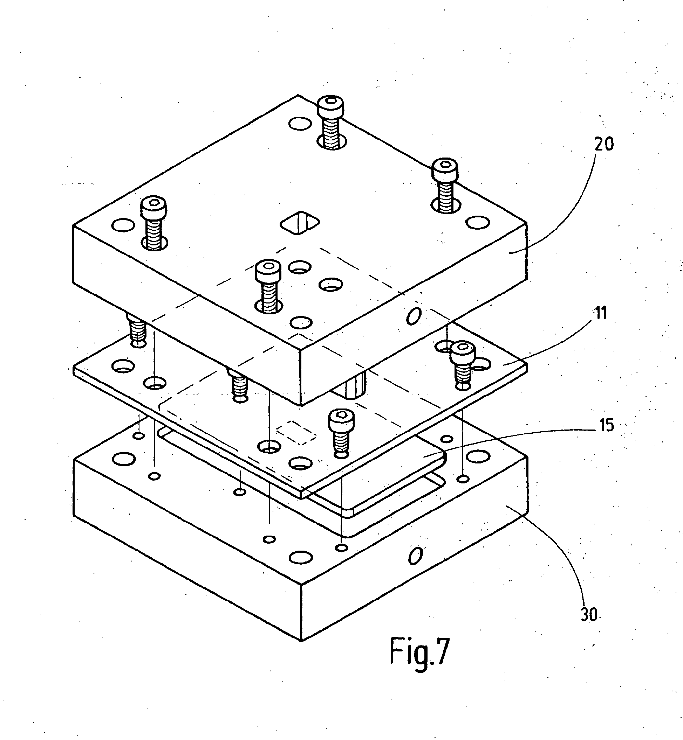

Figur 7 zeigt in einer perspektivischen Ansicht den modularen Aufbau einer oberen Kulturkammer (20), eines Bodenteils (11) mit einer nicht dargestellten Öffnung (5) und einer unteren Kulturkammer (30), in die eine Halterung (15) für die Membran eingepasst werden kann.

### Beispiel 2

### Aufbau eines Testsystems des Dünndarms

Zum Aufbau des Testsystems wurde eine biologische Membran aus porcinem Dünndarm verwendet. Durch eine Behandlung mit Natriumdesoxycholat wurden alle tierischen Zellen von der Membran entfernt. Anschließend wurde die Membran mit PBS gewaschen. Im Folgenden wurde die Membran auf einer Seite mit Endothelzellen und auf der anderen Seite mit CaCo2-Zellen (einer Zelllinie aus dem Darm) besiedelt.

In einer ersten Phase wurde den Zellen erlaubt, auf der Oberfläche der Membran für eine Dauer von ca. 20 Stunden zu adhärieren. Nach der Adhäsion wurde die Membran in den Bioreaktor gemäß Figur 3 eingespannt. Über einen Zeitraum von drei Wochen wurden physiologische Bedingungen aufrechterhalten. Als Medium wurde DMEM (Dulbecco's Modified Eagle Medium) in einem Gesamtvolumen von 20 ml verwendet und alle 3 bis 4 Tage ausgetauscht. In beiden Kulturkammern wurde durch eine jeweils eigene angeschlossene Mikropumpe eine Versorgung mit unterschiedlichem, jeweils zellspezifischem Nährmedium ermöglicht.

Nach dem Zeitraum von drei Wochen wurden Permeationsstudien durchgeführt und dazu eine zu testende Substanz in die Kulturkammer, die den Darmzellen zugewandt ist, gegeben. Durch Probenzug wurde die Permeation ermittelt. Die Diffusionskonstanten lagen in einem Bereich von 10⁻⁶ cm²/s.

## Patentansprüche

1. Bioreaktor (100), umfassend mindestens eine Kulturkammer (20) mit einem Bodenteil (11), wobei das Bodenteil (11) mindestens eine Öffnung (5) aufweist, wobei der Bioreaktor mindestens zwei durch die Öffnung (5) miteinander in Gas- oder Fluidkontakt stehende Kulturkammern (20,30) aufweist, wobei jeder Kulturkammer (20,30) mindestens eine in Gas- oder Fluidkontakt stehende Mikropumpe (40,50) zugeordnet ist, wobei die Kulturkammer (20) eine in der Öffnung (5) angeordnete Membran (10) oder einen Filter aufweist.

2. Bioreaktor (100) nach einem der vorhergehenden Ansprüche, wobei der Bioreaktor modular aufgebaut ist.

3. Bioreaktor nach einem der vorhergehenden Ansprüche, wobei die Kulturkammern (20, 30) und die Mikropumpen (40,50) jeweils als Modul ausgebildet sind.

4. Bioreaktor nach einem der vorhergehenden Ansprüche, wobei die Mikropumpen (40,50) eine funktionskritische Abmessung von 1 bis 800 µm aufweisen.

5. Verfahren zum Kultivieren von Zellen, wobei die Zellen in eine Kulturkammer (20,30) eines Bioreaktors nach einem der Ansprüche 1 bis 4 eingebracht und kultiviert werden.

6. Verfahren nach Anspruch 5, wobei die Zellen auf der Membran (10) in der Kulturkammer (20,30) aufgebracht sind.

7. Verfahren zum Testen von mindestens einer Substanz, wobei die zu testende Substanz in eine Kulturkammer (20,30) eines Bioreaktors (100) nach einem der Ansprüche 1 bis 4 eingebracht und deren Wirkung auf in der Kulturkammer (20,30) befindliche Zellen untersucht wird.

8. Verfahren nach Anspruch 7, wobei die Zellen auf der Membran (10) oder dem Filter des Bioreaktors (100) befindlich sind und die Wirkung der zu testenden Substanz unter aktiver Perfusion der Kulturkammer (20,30) mit einem Kulturmedium durch eine Strömung parallel zu der Membran (10) oder dem Filter untersucht wird.

## Claims

1. A bioreactor (100) comprising at least one culture chamber (20) having a bottom part (11), wherein the bottom part (11) has at least one opening (5), wherein the bioreactor has at least two culture chambers (20, 30) being in gas or fluid contact via the opening (5),wherein each culture chamber (20, 30) is associated with at least one micropump (40, 50) being in gas or fluid contact, wherein the culture chamber (20) having a membrane (10) or a filter located in the opening (5).

2. The bioreactor (100) according to the preceding claim, wherein the bioreactor is of modular construction.

3. The bioreactor according to any one of the preceding claims, wherein the culture chambers (20, 30) and the micropumps (40, 50) are each configured as a module.

4. The bioreactor according to any one of the preceding claims, wherein the micropumps (40, 50) have a function-critical dimension of 1 to 800 µm.

5. A method for cultivating cells, wherein the cells are introduced into and cultivated in a culture chamber (20, 30) of a bioreactor according to any one of claims 1 to 4.

6. The method according to claim 5, wherein the cells are applied to the membrane (10) in the culture chamber (20, 30).

7. A method for testing at least one substance, wherein the substance to be tested is introduced into one culture chamber (20, 30) of a bioreactor (100) according to any one of claims 1 to 4, and its effect on the cells present in the culture chamber (20, 30) is investigated.

8. The method according to claim 7, wherein the cells are present on the membrane (10) or the filter of the bioreactor (100), and the effect of the substance to be tested is investigated under active perfusion of the culture chamber (20, 30) with a culture medium by means of a stream parallel to the membrane (10) or the filter.

## Revendications

1. Bioréacteur (100) comportant au moins une chambre de culture (20) avec une partie de fond (11), la partie de fond (11) présentant au moins une ouverture (5), le bioréacteur présentant au moins deux chambres de culture (20, 30) en contact gazeuse ou fluidique l'une avec l'autre par l'ouverture (5), chaque chambre de culture (20,30) étant associée avec au moins une micropompe (40, 50) en contact gazeuse ou fluidique, la chambre de culture (20) présentant une membrane (10) disposé(e) dans l'ouverture (5) ou un filtre.

2. Bioréacteur (100) selon la revendication précédente, le bioréacteur étant de conception modulaire.

3. Bioréacteur selon l'une quelconque des revendications précédentes, dans lequel les chambres de culture (20, 30) et les micropompes (40, 50) sont respectivement configurées de manière modulaire.

4. Bioréacteur selon l'une quelconque des revendications précédentes, dans lequel les micropompes (40, 50) présentent une dimension essentielle pour le fonctionnement de 1 à 800 µm.

5. Procédé pour cultiver des cellules, dans lequel les cellules sont introduites et cultivées dans une chambre de culture (20, 30) d'un bioréacteur selon l'une quelconque des revendications 1 à 4.

6. Procédé selon la revendication 5, dans lequel les cellules sont appliquées à la membrane (10) dans la chambre de culture (20, 30).

7. Procédé pour tester au moins une substance, dans lequel la substance à tester est introduite dans une chambre de culture (20, 30) d'un bioréacteur (100) selon l'une quelconque des revendications 1 à 4, et ses effets sur les cellules présentes dans la chambre de culture (20, 30) sont examinés.

8. Procédé selon la revendication 7, dans lequel les cellules sont présentes sur la membrane (10) ou sur le filtre du bioréacteur (100), et l'effet de la substance à tester est examinés sous perfusion active de la chambre de culture (20, 30) avec un milieu de culture par écoulement parallèle à la membrane (10) ou au filtre.
